(19) Europäisches Patentamt
European Patent Office
Office européen des brevets

(11) EP 1 319 399 A1

(12) EUROPEAN PATENT APPLICATION
published in accordance with Art. 158(3) EPC

(43) Date of publication:
18.06.2003 Bulletin 2003/25

(21) Application number: 01970145.7

(22) Date of filing: 19.09.2001

(51) Int Cl.[7]: A61K 9/16, A61K 47/10, A61K 47/14, A61K 47/04, A61K 31/4365, A61K 31/497

(86) International application number:
PCT/JP01/08137

(87) International publication number:
WO 02/024167 (28.03.2002 Gazette 2002/12)

(84) Designated Contracting States:
AT BE CH CY DE DK ES FI FR GB GR IE IT LI LU MC NL PT SE TR
Designated Extension States:
AL LT LV MK RO SI

(30) Priority: 19.09.2000 JP 2000283565

(71) Applicant: DAIICHI PHARMACEUTICAL CO., LTD.
Chuo-ku, Tokyo 103-8234 (JP)

(72) Inventors:
- KIKUCHI, H. DAIICHI PHARMACEUTICAL CO. LTD R&D CT
Tokyo 134-8630 (JP)
- IKETANI, M. DAIICHI PHARMACEUTICAL CO. LTD R&D CT
Tokyo 134-8630 (JP)
- KOBAYASHI, H. DAIICHI PHARMAC. CO. LTD R&D CT
Tokyo 134-8630 (JP)

(74) Representative: Hartz, Nikolai F., Dr. et al
Wächtershäuser & Hartz,
Patentanwälte,
Tal 29
80331 München (DE)

# (54) MEDICINAL COMPOSITION

(57) A pharmaceutical composition containing a drug (A), a waxy substance (B), and synthetic aluminum silicate and/or hydrous silicon dioxide (C). The invention provides a granular pharmaceutical composition suitable for providing a pharmaceutical characterized in that adhesion of granules thereof onto a granulation apparatus during granulation is minimized and caking of the granules is suppressed.

EP 1 319 399 A1

## Description

Technical Field

The present invention relates to a pharmaceutical composition which does not cause adhesion of granules thereof onto a granulation apparatus during granulation and which prevents caking of the granules.

Background Art

Regarding pharmaceutical preparations having sustained drug effects (sustained-release pharmaceutical preparations) and pharmaceutical preparations which mask a disagreeable taste of drug, there have been known granular pharmaceutical preparations which have been prepared by dissolving or dispersing a drug in a molten waxy substance and spray-granulating the resultant solution or dispersion by use of a spray drier or similar means (disclosed in, for example, Japanese Patent Application Laid-Open (*kokai*) Nos. 2-275817 and 7-242568).

However, they involve the problem that, during spray granulation of a molten waxy substance in which a drug is dissolved or dispersed, the granulated product adheres onto the inner wall or other parts of a spray granulation apparatus, resulting in a considerable decrease in yield of the granulated product. In addition, the thus-obtained granulated product disadvantageously undergoes aggregation of granules; i.e., caking.

Disclosure of the Invention

The present inventors have carried out extensive studies on a variety of substances which may be effective to prevent adhesion of the aforementioned granulated product, and have found that, by adding synthetic aluminum silicate and/or hydrous silicon dioxide during spray granulation, adhesion of the granulated product onto the inside of a granulation apparatus can be remarkably prevented, attaining the enhancement of the yield of the granulated product, and that pharmaceutical products obtained from the granulated product possess excellent characteristics. The inventors have also found that caking of the thus-obtained granulated product can be prevented, and that, even if caking occurs, the granulated product can be readily disaggregated. The present invention has been accomplished on the basis of these findings.

Accordingly, the present invention provides a pharmaceutical composition comprising a drug (A), a waxy substance (B), and synthetic aluminum silicate and/or hydrous silicon dioxide (C), as well as an oral pharmaceutical preparation containing the composition.

The present invention also provides an agent for preventing adhesion of a granulated product onto the wall inside a granulation apparatus during spray granulation, the agent containing, as an effective ingredient, synthetic aluminum silicate and/or hydrous silicon dioxide.

Best Modes for Carrying Out the Invention

No particular limitation is imposed on the drug (A) to be employed in the present invention, so long as the drug is used as a pharmaceutical agent. Since the pharmaceutical composition of the present invention exerts an effect of masking a disagreeable taste of a drug, preferably, the drug to be employed in the composition has a disagreeable taste.

In the present invention, the term "disagreeable taste" refers to such a taste that persons receive, upon taking a drug in the mouth, a sensation including a bitter taste, an astringent effect, a pungent taste, a stimulation, and an odor. Examples of the drug providing the disagreeable taste include cetraxate hydrochloride, ecapapide, nefiracetam, talampicillin hydrochloride, indenolol hydrochloride, hydralazine hydrochloride, chloropromazine hydrochloride, tiaramide hydrochloride, berberine chloride, digitoxin, sulpyrine, azelastine hydrochloride, etilefurine hydrochloride, diltiazem hydrochloride, propranolol hydrochloride, chloramphenicol, aminophyllin, erythromycin, clarithromycin, phenobarbital, calcium pantothenate, indeloxazine hydrochloride, aminoguanidine hydrochloride, bifemelane hydrochloride, 7β-[2-(2-aminothiazol-4-yl)-2-(Z)-hydroxyiminoacetamido]-3-N,N-dimethylcarbamoyloxymethyl-3-cephem-carboxylic acid 1-(isopropoxycarbonyloxy)ethyl ester hydrochloride, (E)-3-(2-methoxy-3,6-dimethyl-1,4-benzoquinon-5-yl)-2-[5-(3-pyridyl)pentyl]-2-propenic acid, aminophylline, theophylline, diphenhydramine, metoclopramide, phenylbutazone, phenobarbital, ampicillin, cimetidine, famotidine, nizatidine, acetoaminophene, epirizol, pyrazinamide, caffeine, ethionamide, carbezirol, ranitidine hydrochloride, roxatidine acetate hydrochloride, imipramine hydrochloride, ephedrine hydrochloride, diphenhydramine hydrochloride, donepezil hydrochloride, tetracycline hydrochloride, doxycycline hydrochloride, naphazoline hydrochloride, noscapine hydrochloride, papaverine hydrochloride, dextrometorphan hydrobromide, timepidium bromide, chlorophenylammonium maleate, alimemazine tartrate, pilsicainide hydrochloride, N-methylscopolammonium methylsulfate, cinepazide maleate, arginine hydrochloride, hystidine hydrochloride, lysine hydrochlroride, lysine acetate; crude drugs or extracts thereof such as *Papaveraceae, Rutaceae, Ranunculaceae, Nux vomica, Ephedraceae, Rubiaceae, Solanaceae, belladonna,* or *Reguminsae*; pyrridonecarboxylic acid compounds represented by formulas (1) through (4) and salts thereof:

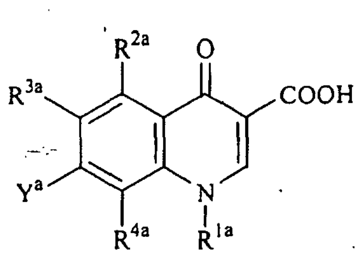

(1)

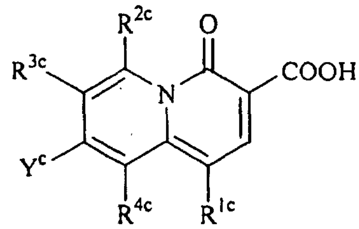

(2)

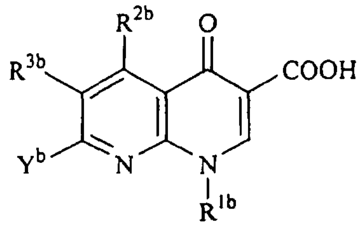

(3)

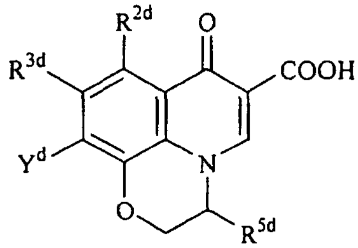

(4)

(wherein each of $R^{1a}$, $R^{1b}$, and $R^{1c}$ represents a C1-C6 linear or branched alkyl group which may have a substituent, a C3-C6 cyclic alkyl group which may have a substituent, an aryl group which may have a substituent, or a heteroaryl group which may have a substituent;
each of $R^{2a}$, $R^{2b}$, $R^{2c}$, and $R^{2d}$ represents a hydrogen atom a C1-C6 linear or branched alkyl group which may have a substituent or an amino group;
each of $R^{3a}$, $R^{3b}$, $R^{3c}$, and $R^{3d}$ represents a hydrogen atom or a halogen atom;
$R^{4a}$ or $R^{4c}$ represents a hydrogen atom, a halogen atom, a C1-C6 linear or branched alkyl group which may have a substituent;
or a C1-C6 linear or branched alkoxyl group which may have a substituent;
$R^{5d}$ represents a hydrogen atom or a C1-C6 linear or branched alkyl group which may have a substituent; and
each of $Y^a$, $Y^b$, $Y^c$, and $Y^d$ represents a nitrogen-containing group); and
4,5,6,7-tetrahydrothieno[3,2-c]pyridines or salts thereof represented by formula (5):

$$R^1\text{-CH}(R^2)\text{-}R^3 \qquad (5)$$

[wherein $R^1$ represents a phenyl group which may have 1 to 3 substituents selected from the group consisting of a C1-C4 alkyl group, a halogen atom, a fluorine-substituted C1-C4 alkyl group, a C1-C4 alkoxy group, a fluorine-substituted C1-C4 alkoxyl group, a cyano group, and a nitro group;
$R^2$ represents a hydrogen atom, a carboxyl group, a C1-C6 alkoxycarbonyl group, or a C1-C7 aliphatic acyl group which may have a substituent selected from among a halogen atom, a hydroxyl group, a C1-C6 alkoxyl group, and a cyano group; and
$R^3$ represents a 4,5,6,7-tetrahydrothieno[3,2-c]pyridin-5-yl, group which may have a substituent selected from among a hydroxyl group, a C1-C4 alkoxyl group, a C1-C4 alkoxyl group which are substituted by C1-C4 alkoxyl or C1-C6 alkanoyloxy, a C7-C14 aralkyloxy group, a C1-C18 alkanoyloxy group, a C3-C7 cycloalkylcarbonyloxy group, a C6-C10 arylcarbonyloxy group, a C1-C4 alkoxycarbonyloxy group, and a C7-C14 aralkyloxycarbonyloxy group].

The above-described pyrridonecarboxylic acid compounds represented by formulas (1), (2), (3), or (4) and salts thereof are described in the following references: Japanese Patent Application Laid-Open (*kokai*) Nos. 53-141286,

55-31042, 57-46986, 57-77683, 60-36482, 60-64979, 60-228479, 62-252772, 62-252790, 62-277362, 1-230558, 1-258666, 1-294680, 2-28178, 2-124873, 2-231475, 5-271229, 7-309864, 8-41050 and WO 91/02526, WO 94/14794, WO 94/15933, WO 95/5373, WO 96/37475, WO 96/39407, WO 97/29102, WO 97/19072, WO 97/40037, WO 98/02431, WO 98/13370, WO 98/18783, WO 98/24781, WO 98/52939, WO 98/54169, and WO 98/58923. These publications also disclose production methods of the compounds and salts.

The compounds represented by formula (5) and salts thereof may be produced by a method described in Japanese Patent Application Laid-Open (*kokai*) Nos. 50-46688, 58-10583, 59-27895, and 6-41139.

Any of the above-described compounds represented by formulas (1), (2), (3), (4), or (5) may have an asymmetric carbon atom and may exist as an optical isomer or a diastereomer. Such isomers *per se,* arbitrary mixtures thereof, racemic species, etc. are encompassed within the scope of the present invention. The above-described compounds represented by formulas (1) through (5) may exist as salts thereof, hydrates thereof, or solvates thereof, which are also included within the scope of the present invention.

In view of effect for masking a disagreeable taste, the drug (A) of the present invention is preferably slightly soluble in the waxy substance (B); more preferably, soluble in water and slightly soluble in the waxy substance (B).

Among the above-described compounds represented by formulas (1) through (4) and salts thereof, examples of preferred compounds include the following:

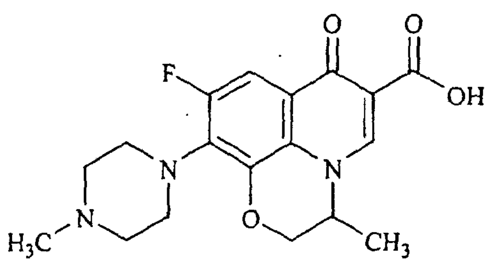

Ofloxacin

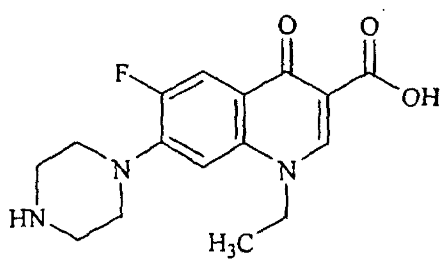

Norfloxacin

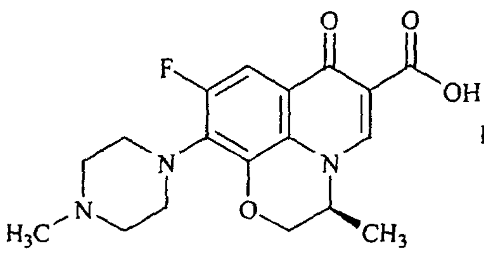

Levofloxacin

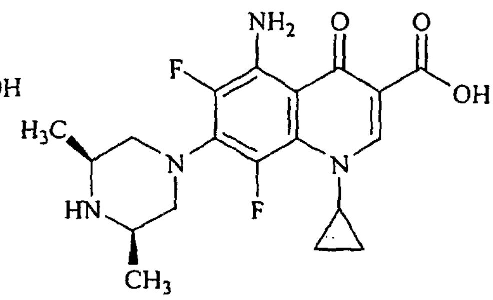

Sparfloxacin

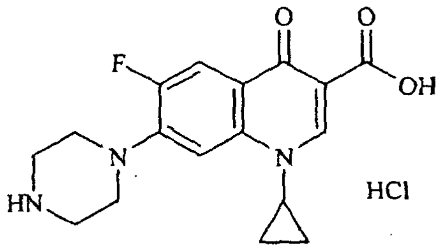

Ciprofloxacin hydrochloride

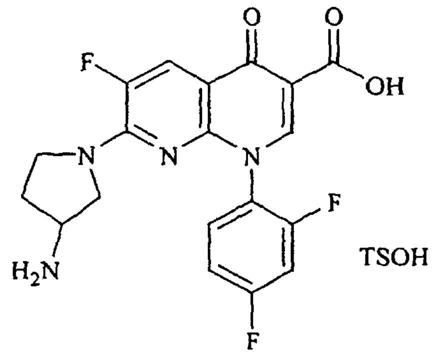

Tosufloxacin tosilate

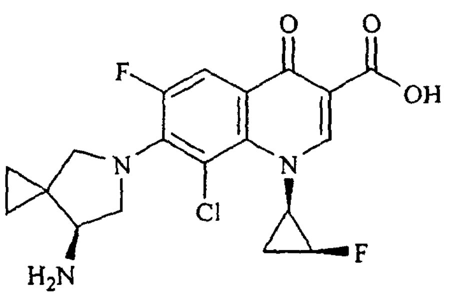

Sitafloxacin

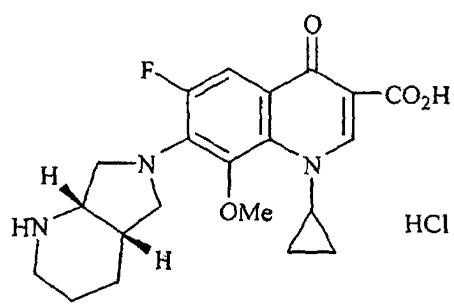

Moxifloxacin hydrochloride

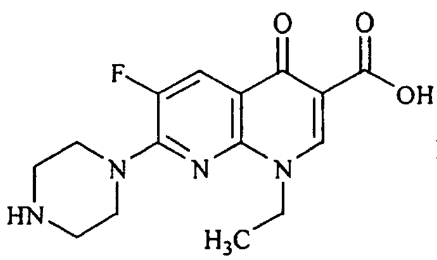

Enoxacin

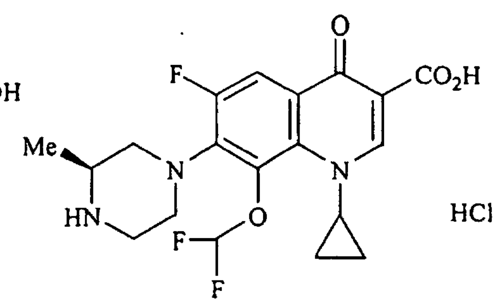

CS-940

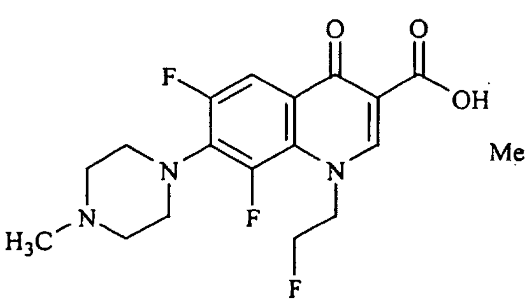

Fleroxacin

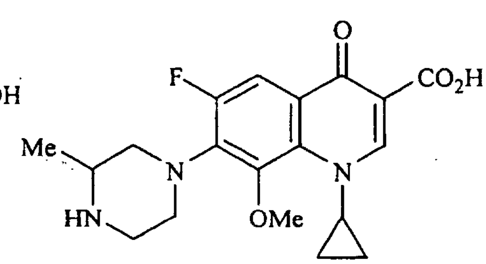

Gatifloxacin

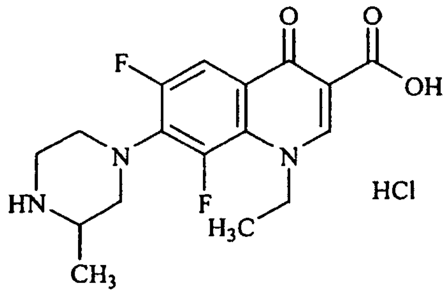

Lomefloxacin hydrochloride

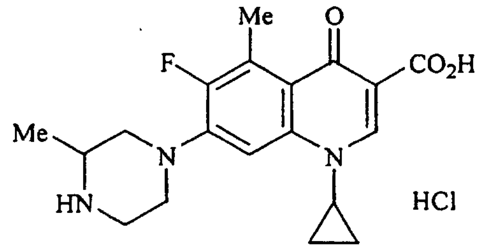

Grepafloxacin hydrochloride

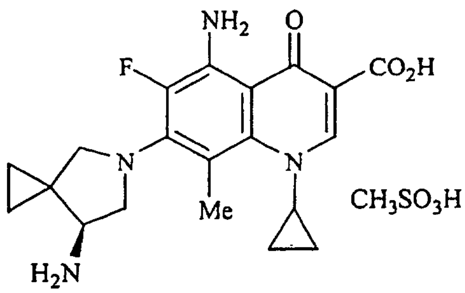

HSR-903

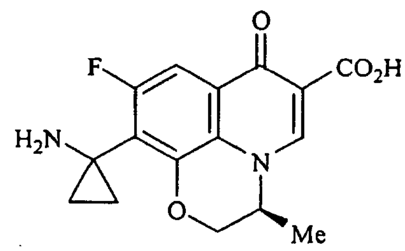

Pazufloxacin

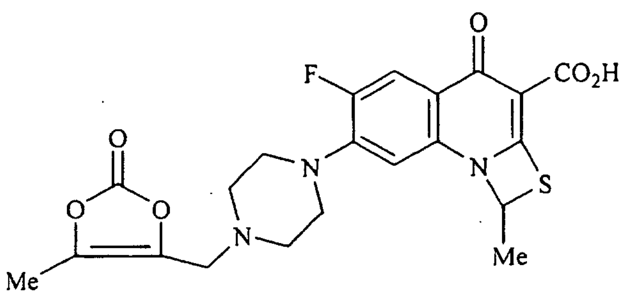

Prulifloxacin

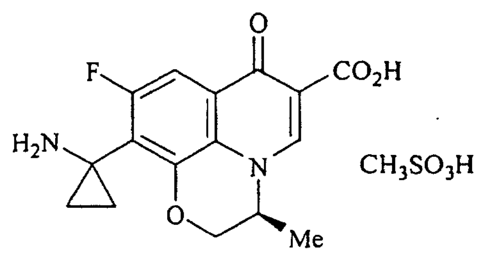

Pazufloxacin mesilate

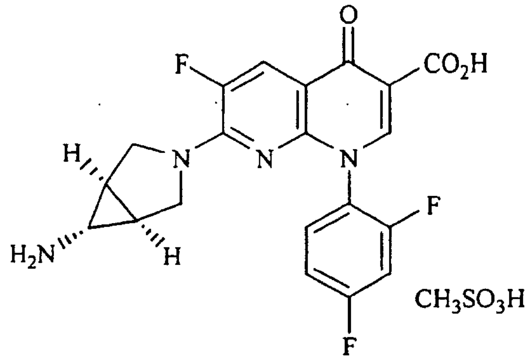

Trovafloxacin mesilate

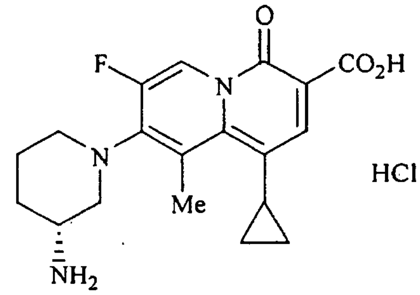

A-99058.L

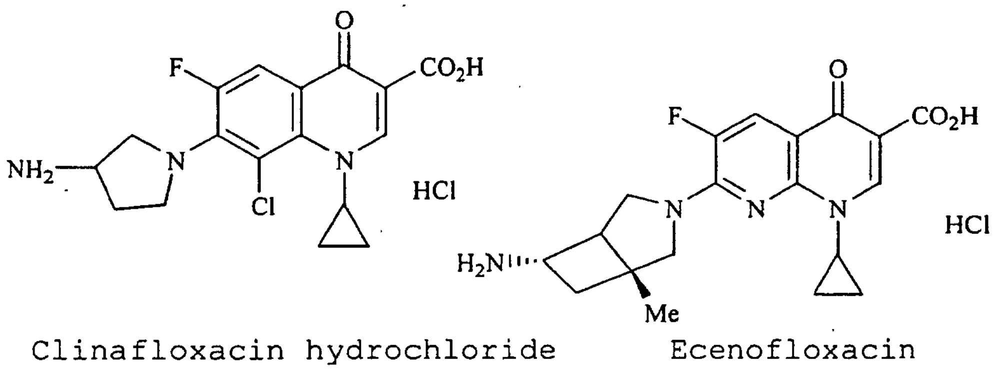

HCl HCl

Clinafloxacin hydrochloride Ecenofloxacin

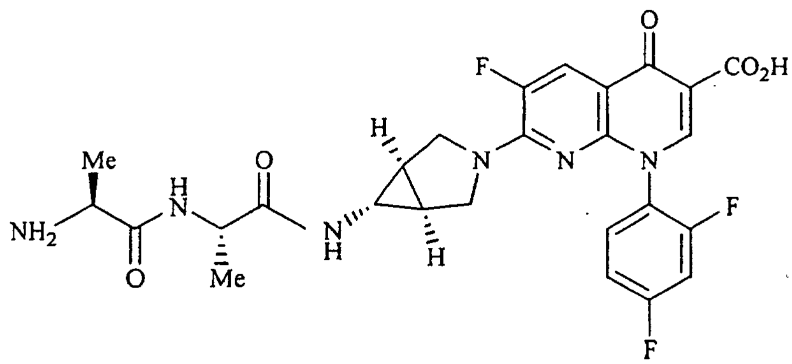

$CH_3SO_3H$

Alatrofloxacin mesilate

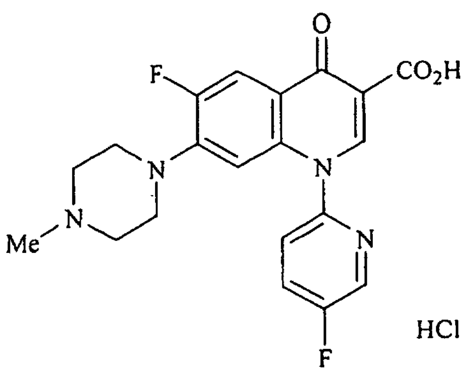

HCl

Fandofloxacin hydrochloride

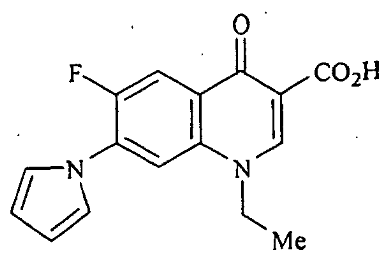

Irloxacin

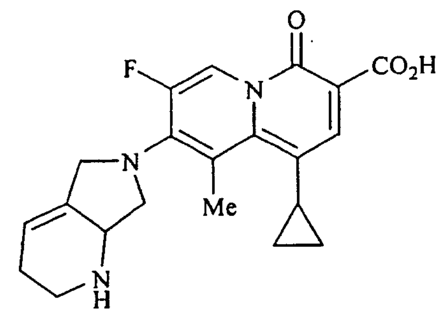

KRQ-10018

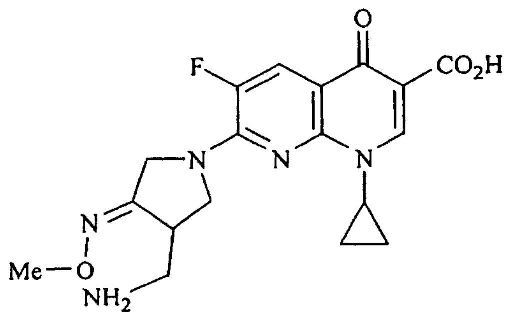

LB-20304a

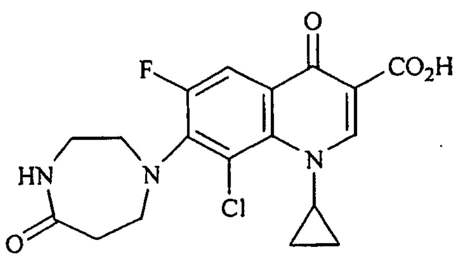

SS-732

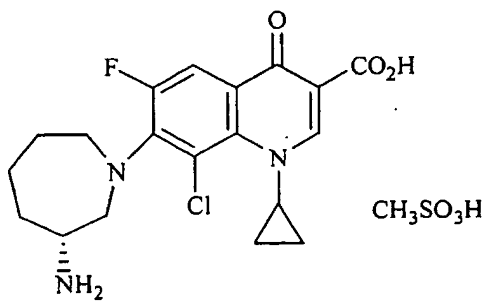

$CH_3SO_3H$

SS-734

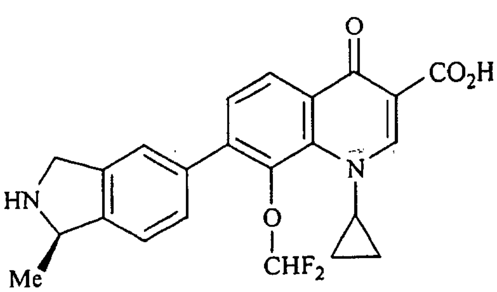

T-3811

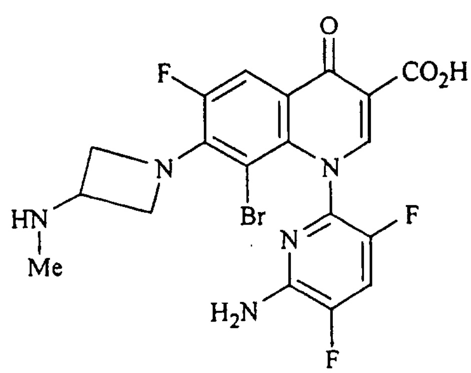

WQ-2743

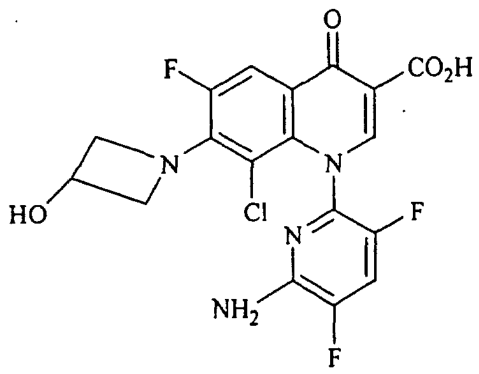

WQ-3034

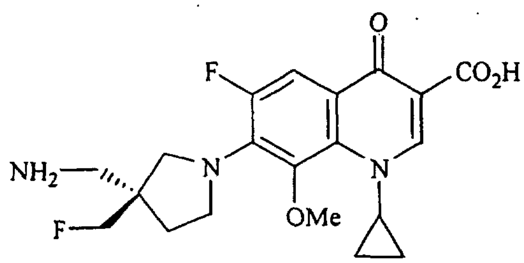

Y-688

Also, among the compounds represented by formula (5) and salts thereof, examples of preferred compounds include the following:

2-hydroxy-5-(α-cyclopropylcarbonyl-2-chlorobenzyl)-4,5,6,7-tetrahydrothieno[3,2-c]pyridine,
2-hydroxy-5-(α-propionyl-2-fluorobenzyl)-4,5,6,7-tetrahydrothieno[3,2-c]pyridine,
2-hydroxy-5-(α-cyclopropylcarbonyl-2-fluorobenzyl)-4,5,6,7-tetrahydrothieno[3,2-c]pyridine,
2-acetoxy-5-(α-cyclopropylcarbonyl-2-fluorobenzyl)-4,5,6,7-tetrahydrothieno[3,2-c]pyridine,
2-propionyloxy-5-(α-cyclopropylcarbonyl-2-fluorobenzyl)-4,5,6,7-tetrahydrothieno[3,2-c]pyridine,
2-butyryloxy-5-(α-cyclopropylcarbonyl-2-fluorobenzyl)-4,5,6,7-tetrahydrothieno[3,2-c]pyridine,
2-pivaloyloxy-5-(α-cyclopropylcarbonyl-2-fluorobenzyl)-4,5,6,7-tetrahydrothieno[3,2-c]pyridine,
2-valeryloxy-5-(α-cyclopropylcarbonyl-2-fluorobenzyl)-4,5,6,7-tetrahydrothieno[3,2-c]pyridine,
2-hexanoyloxy-5-(α-cyclopropylcarbonyl-2-fluorobenzyl)-4,5,6,7-tetrahydrothieno[3,2-c]pyridine,
2-t-butoxycarbonyloxy-5-(α-cyclopropylcarbonyl-2-fluorobenzyl)-4,5,6,7-tetrahydrothieno[3,2-c]pyridine,
2-pivaloyloxymethoxy-5-(α-cyclopropylcarbonyl-2-fluorobenzyl)-4,5,6,7-tetrahydrothieno[3,2-c]pyridine,
5-(α-cyclopropylcarbonyl-2-chlorobenzyl)-2-oxo-2,4,5,6,7,7a-hexahydrothieno[3,2-c]pyridine,
5-(α-propionyl-2-fluorobenzyl)-2-oxo-2,4,5,6,7,7a-hexahydrothieno[3,2-c]pyridine,
5-(α-cyclopropylcarbonyl-2-fluorobenzyl)-2-oxo-2,4,5,6,7,7a-hexahydrothieno[3,2-c]pyridine,
2-acetoxy-5-(α-cyclopropylcarbonyl-2-chlorobenzyl)-4,5,6,7-tetrahydrothieno[3,2-c]pyridine,
2-hydroxy-5-(α-2-fluorocyclopropylcarbonyl-2-fluorobenzyl)-4,5,6,7-tetrahydrothieno[3,2-c]pyridine,
5-(α-2-fluorocyclopropylcarbonyl-2-fluorobenzyl)-2-oxo-2,4,5,6,7,7a-hexahydrothieno[3,2-c]pyridine,
2-acetoxy-5-(α-2-fluorocyclopropylcarbonyl-2-fluorobenzyl)-4,5,6,7-tetrahydrothieno[3,2-c]pyridine,
5-(α-methoxycarbonyl-2-chlorobenzyl)-2-oxo-2,4,5,6,7,7a-hexahydrothieno[3,2-c]pyridine,
2-acetoxy-5-(α-methoxycarbonyl-2-chlorobenzyl)-4,5,6,7-tetrahydrothieno[3,2-c]pyridine,
5-(α-methoxycarbonyl-2-fluorobenzyl)-2-oxo-2,4,5,6,7,7a-hexahydrothieno[3,2-c]pyridine,
2-acetoxy-5-(α-methoxycarbonyl-2-fluorobenzyl)-4,5,6,7-tetrahydrothieno[3,2-c]pyridine,
5-(2-chlorobenzyl)-4,5,6,7-tetrahydrothieno[3,2-c]pyridine (nonproprietary name: ticlopidine; available as ticlopidine hydrochloride),
5-(α-methoxycarbonyl-2-chlorobenzyl)-4,5,6,7-tetrahydrothieno[3,2-c]pyridine (nonproprietary name: clopidogrel; available as clopidogrel sulfate),
5-(α-methoxycarbonyl-2-fluorobenzyl)-4,5,6,7-tetrahydrothieno[3,2-c]pyridine,
5-(α-cyclopropylcarbonyl-2-chlorobenzyl)-4,5,6,7-tetrahydrothieno[3,2-c]pyridine,
5-(α-cyclopropylcarbonyl-2-fluorobenzyl)-4,5,6,7-tetrahydrothieno[3,2-c]pyridine,
5-(α-propionyl-2-chlorobenzyl)-4,5,6,7-tetrahydrothieno[3,2-c]pyridine, and
5-(α-propionyl-2-fluorobenzyl)-4,5,6,7-tetrahydrothieno[3,2-c]pyridine; and salts thereof.

In the present invention, the drug (A) is preferably ofloxacin, levofloxacin, cytafloxacin hydrate, cetraxate hydrochloride, nefiracetam, ticlopidine hydrochloride, or clopidogrel sulfate.

Examples of the waxy substance (B) (specifically, a waxy substance having a melting point of 40-150°C) which is used in the present invention include fats and oils such as hydrogenated oils (e.g., hydrogenated castor oil, hydrogenated soy bean oil, hydrogenated rape seed oil, hydrogenated cotton seed oil) and fats and oils of vegetable or animal origin (e.g., carnauba wax, white beeswax, beef tallow); alcohols and polyhydric alcohols such as higher

alcohols (e.g., stearyl alcohol, cetanol) and polyethylene glycol (e.g., Macrogol 4000, Macrogol 6000); fatty acids and derivatives thereof such as higher fatty acids (e.g., stearic acid, palmitic acid) and fatty acid glycerin esters (e.g., fatty acid glycerin monoester, fatty acid glycerin triester) and fatty acid sucrose esters; and mixtures of two or more of these substances. Of these, hydrogenated oils, fatty acids, and derivatives of fatty acids are preferred; with hydrogenated oils, higher fatty acids, and fatty acid esters being more preferred; and hydrogenated oils, fatty acid glycerin monoesters, fatty acid glycerin triesters, and stearic acid being particularly preferred. From the viewpoint of the effect of masking the disagreeable taste of the drug (A), the waxy substance preferably has a melting point lower than that of the drug.

In the present invention, synthetic aluminum silicate and/or hydrous silicon dioxide (C) serves as the agent for preventing adhesion of a granulated product onto the wall inside a granulation apparatus during spray granulation. When synthetic aluminum silicate or hydrous silicon dioxide is used, adhesion of a granulated product onto the wall inside a granulation apparatus can remarkably be suppressed, as compared with the case in which any of other ingredients employable as additives for pharmaceutical preparations is used. Thus, production efficiency for granulated products is drastically enhanced through addition of component (C). In contrast, granulated products obtained without adding component (C) to the composition undergo strong caking, and an additional operation is required for disaggregating the formed cakes during the production process. Since granulated products obtained from the composition containing component (C) cause no caking or, even when caking occurs, the granulated products can be readily separated, no additional operation for disaggregating cakes during the production process is required, leading to enhancement of operation efficiency.

In addition, the pharmaceutical composition of the present invention to which component (C) has been added exhibits an excellent drug release property upon dissolution as well as excellent effect for masking a disagreeable taste of the drug (A).

In the present invention, the ratio by weight of the drug (A) to the waxy substance (B) is preferably 1 : 1 to 1 : 5 from the viewpoints of the balance between effect of masking a disagreeable taste and the drug-release property. Synthetic aluminum silicate and hydrous silicon dioxide may be used in combination to provide component (C). The component (C) is incorporated into the pharmaceutical composition of the present invention preferably in an amount of 0.1-5 wt.%, particularly preferably 0.5-4 wt.%, further preferably 1-4 wt.%, in view of the aforementioned adhesion prevention effect and caking prevention effect.

The effect for masking a disagreeable taste and the sensation upon oral administration of the pharmaceutical composition of the present invention can be enhanced by further adding sugar alcohol into the composition. Sugar alcohols having low heat of dissolution are preferred; for example, erythritol, xylitol, maltitol, or a mixture of two or more of these compounds. From the viewpoint of sensation upon oral administration, a sugar alcohol having a heat of dissolution of -30 cal/g or lower is preferred, and erythritol and xylitol are particularly preferred. The percentage of sugar alcohol in the pharmaceutical composition of the invention is preferably 10 wt.% or higher, more specifically 10-99.9 wt.%, more preferably 20-80 wt.%, most preferably 30-70 wt.% from the viewpoints of masking effect, drug-release property, and sensation upon oral administration.

The pharmaceutical composition of the present invention may be prepared as follows. The waxy substance (B) is melted with heat, and the drug (A), synthetic aluminum silicate and/or hydrous silicon dioxide (C), and an optional component are dispersed or dissolved therein. Subsequently, the resultant dispersion or solution is subjected to spray granulation.

In a preferred manner of spray granulation, the aforementioned dispersion or solution is added dropwise to a rotary disk rotating in a general spray granulation apparatus. The disk is caused to rotate preferably at high speed; i.e., generally at 200-30,000 rpm, preferably 500-25,000 rpm. The speed of addition (feeding) of the dispersion or solution to the rotary disk is appropriately determined in consideration of rotation speed of the disk or other factors. Preferably, the speed of addition is typically 2 g/min to 300 g/min, particularly 5 g/min to 200 g/min.

The rotary disk may be, in terms of shape, of a pin type, vane type, or Kestner type. Of these, a pin type is preferred.

The thus-obtained granulated product containing drug (A), waxy substance (B), and synthetic aluminum silicate and/or hydrous silicon dioxide (C) may be used as the pharmaceutical composition without undergoing any further treatment. Alternatively, the product may be subjected to further secondary granulation.

Secondary granulation may be accomplished by wet fluidized bed granulation, wherein a binder solution such as a solution of hydroxypropylcellulose, hydroxypropylmethylcellulose, polyvinylpyrrolidone, or sorbitol is used. Alternatively, secondary granulation may be accomplished by melting granulation, wherein a low-melting-point substance such as polyethylene glycol or glycerin monostearate is used as a binder.

In the case in which the aforementioned secondary granulation is performed, the aforementioned sugar alcohol is added preferably at the stage of secondary granulation. Briefly, in the mouth, sugar alcohol employed in secondary granulation is dissolved in saliva in approximately ten seconds, leaving only the waxy substance particles containing the drug in the form of a dispersion and obtained through primary granulation. However, since particles of the waxy substance having a particle size of 50-200 μm are fine spheres, no disagreeable, foreign sensation to the

mouth is provided. Furthermore, there can be formed particles in which the drug is dispersed uniformly in a waxy substance, to thereby achieve successful masking of the drug's disagreeable taste, because the very low amount of the drug is dissolved in the mouth. Sugar alcohols, particularly erythritol and xylitol, taste sweet and deliver fresh and cool sensation to the mouth, yielding the effect of masking the drug's disagreeable taste. After being swallowed, the waxy substance particles release the drug in the digestive tract, resulting in absorption of the released drug into the body.

The pharmaceutical composition of the present invention may be prepared-with or without addition of other additives according to needs-into pharmaceutical products for oral administration, such as powders, fine granules, granules, dry syrups, tablets, and capsules. Particularly, powders, fine granules, granules, and dry syrups are preferred.

Examples of the aforementioned additives to be added to the composition include binders such as polyvinylpyrrolidone, polyvinyl alcohol, hydroxypropylcellulose, hydroxypropylmethylcellulose, methylcellulose, polyethylene glycol, glycerin monostearate, and sorbitol; sweeteners such as aspartame, saccharin sodium, saccharin, sodium saccharate, saccharin, thumatin, and stevia; aromatic ingredients such as dl-menthol, l-menthol, and Menthol micron; fluidizing agents such as light anhydrous silicic acid, magnesium metasilicate aluminate, talc, and ethylcellulose; disintegrants such as crosscarmellose sodium, sodium starch gluconate, and low substituted hydroxypropylcellulose; and pH regulators such as sodium citrate and sodium bicarbonate.

[Examples]

The present invention will next be described in more detail by way of examples, which should not be construed as limiting the invention thereto.

Example 1

Glycerin monoisostearate (209.87 g) was melted at about 90°C, and any of synthetic aluminum silicate, hydrous silicon dioxide, light anhydrous silicic acid, olive oil, propylene glycol, silicone resin, talc, or triacetyl glycerin (3.13 g) was added to the melt. In the resultant mixture, ticlopidine hydrochloride (100 g) was uniformly dispersed. The dispersion was subjected to spray granulation by use of a spray drier to thereby obtain granules.

The spray granulation was performed under the following conditions.

(Spray Granulation Conditions)

Apparatus: Spray drier (Model L-8, diameter: 80 cm, product of Okawara Kakoki)
Rotary disk: MC-50 (product of Okawara Kakoki)
Diameter of rotary disk: 50 mm
Rotation speed of rotary disk: 12,400-12,600 rpm
Feed (add) rate: 28-31 g/min
Intake air temperature: 49.6-50.3°C (about 50°C)

The amount of granules adhered on the wall inside the granulation apparatus was measured. As compared with the case in which no additive other than glycerin monostearate and ticlopidine hydrochloride had been added, a decrease in amount of adhered granules was observed only in the case in which synthetic aluminum silicate or hydrous silicon dioxide had been added. When any of other additives had been added, as compared with additive-free cases, the amount of adhered granules was not reduced.

Example 2

Among the granule samples obtained in Example 1, samples which had been obtained by adding synthetic aluminum silicate or hydrous silicon dioxide were subjected to particle size distribution measurement, dissolution test, and bitter-taste-masking test.

(1) The particle size distribution was measured by means of a laser-diffraction-type particle size distribution measurement apparatus (product of HELOS & RODOS). In both cases, 50% the formed granules had a particle size of 100 μm (volume mediane diameter).
(2) Dissolution test was performed through a paddle method (50 rpm, purified water 900 mL, 37°C). In both cases, the formed granules exhibited excellent drug release property upon dissolution (i.e., substantially complete release

of the drug therein within 30 minutes), showing employability for pharmaceutical products.

(3) Bitter-taste-masking test was performed upon 30-second-dissolution through a paddle method (100 rpm, purified water 300 mL, 37°C). In both cases, the formed granules release only 4.4-5.2% the contained drug for 30-second-dissolution, showing satisfactory effect of masking a bitter taste in the mouth.

Example 3

Glycerin monostearate (203.61 parts by weight) was melted at about 90°C, and synthetic aluminum silicate (9.39 parts by weight) was mixed with the melt. In the resultant mixture, ticlopidine hydrochloride (100 parts by weight) was uniformly dispersed. The dispersion was subjected to spray granulation by use of a spray drier to thereby obtain granules.

In the course of granulation, the amount of adhered granules was small. The formed granules exhibit an excellent particle size distribution, drug release property upon dissolution, and bitter-taste-masking property.

Example 4

Glycerin monostearate (203.7 parts by weight) was melted at about 90°C, and synthetic aluminum silicate (9.3 parts by weight) was mixed with the melt. In the resultant mixture, ticlopidine hydrochloride (100 parts by weight) was uniformly dispersed. The dispersion was subjected to spray granulation by use of a spray drier to thereby obtain minute granules. Erythritol (526 parts by weight) was added to the granules (313 parts by weight) and the mixture was mixed by use of a fluidized-bed granulator. Subsequently, aqueous D-sorbitol solution (68 w/w%) in an amount equivalent to 100 parts by weight of D-sorbitol was sprayed onto the mixture for fluidized-bed granulation. After spraying, the granules were dried in the fluidized-bed granulator to thereby obtain granules. The granules (939 parts by weight) was mixed with light anhydrous silicic acid (45 parts by weight), talc (15 parts by weight), and Menthol micron (1 part by weight), to thereby yield a powder.

Example 5

A powder mixture containing glycerin monostearate (203.7 parts by weight), synthetic aluminum silicate (9.3 parts by weight), and levofloxacin (100 parts by weight) was heated at 90-100°C, to thereby melt glycerin monostearate. The resultant liquid was mixed and subjected to spray granulation by use of a spray drier (having a hollow cylindrical top portion and a conical bottom portion) to thereby obtain granules.

The spray granulation was performed under the following conditions.

(Spray Granulation Conditions)

Apparatus: Spray drier (Model L-8, diameter: 80 cm, product of Okawara Kakoki)
Rotary disk: MC-50 (product of Okawara Kakoki)
Diameter of rotary disk: 50 mm
Rotation speed of rotary disk: about 20,000 rpm
Feed (add) rate: about 50 g/min
Intake air temperature: about 50°C

Referential Example 1

A powder mixture containing glycerin monostearate (213 parts by weight) and levofloxacin (100 parts by weight) was heated at 90-100°C, to thereby melt glycerin monostearate. The resultant liquid was mixed and subjected to spray granulation by use of a spray drier to thereby obtain granules.

The spray conditions employed were identical to those employed in Example 5.

Evaluation (1)

In Example 5 and Referential Example 1, the condition of adhesion of granules on the wall inside the granulator can after completion of granulation was observed.

Table 1 shows the results.

Table 1

| Observation of the wall inside the can (after granulation) | | |
|---|---|---|
| | Example 5 | Ref. Ex. 1 |
| Cylinder portion | Adhesion of a few granules observed | Adhesion of granules observed |
| Conical portion | Adhesion of very few granules observed | Adhesion of granules observed |

As is clear from Table 1, the amount of adhered granules in Example 5 definitely has decreased as compared with the case of Referential Example 1. Thus, the effect of preventing adhesion of granules on the wall inside the granulator can through addition of synthetic aluminum silicate was confirmed.

Evaluation (2)

The percent adhesion of granules on the wall inside the can during granulation was calculated from the following formula.

$$\text{percent adhesion (\%)} = [(\text{amount of granules adhered on the wall inside the can) (g)}/(\text{total amount of recovered granules) (g)}] \times 100$$

Table 2 shows the results on percent adhesion.

Table 2

| Evaluation results on adhesion of granules on the wall inside the can | | |
|---|---|---|
| | Example 5 | Ref. Ex. 1 |
| Percent adhesion | 1.4% | 3.6% |

The percent adhesion obtained in Example 5 was smaller than that obtained in Referential Example 1. Thus, the effect of preventing adhesion of granules on the wall inside the granulator can through addition of synthetic aluminum silicate was confirmed.

Evaluation (3)

Each (about 80 g) of granule sample of Example 5 and that of Referential Example 1 was placed in a glass bottle and stored at room temperature for one day. After completion of storage, occurrence of caking was checked, and caking was observed in both samples. The caked granules were transferred to a sieve (No. 12; mesh size: 1400 μm), and the edge of the sieve was tapped by use of a metal-made spatula. The number of tapping that was required for allowing the entirety of caked granules to pass through the sieve by disaggregation was measured. The number served as an index of caking strength.

Table 3 shows the results of caking strength of granule samples.

Table 3

| Evaluation results of caking strength of granule samples | | |
|---|---|---|
| | Example 5 | Ref. Ex. 1 |
| Number of tapping | 100 | 400 |

The number of tapping required in Example 5 was smaller than that in Referential Example 1. Thus, the effect of improving flowability of granules through addition of synthetic aluminum silicate was confirmed.

Example 6

Glycerin monostearate (203.6 parts by weight) was melted at 90-100°C. After confirmation of melting glycerin

monostearate, synthetic aluminum silicate (9.4 parts by weight) and ticlopidine hydrochloride (100 parts by weight) were added to the resultant liquid. The resultant mixture liquid was subjected to spray granulation by use of a spray drier to thereby obtain granules.

The spray granulation was performed under the following conditions.

(Spray Granulation Conditions)

Apparatus: Spray drier (Model OC-16, diameter: 160 cm, product of Okawara Kakoki)
Rotary disk: MC-65 (product of Okawara Kakoki)
Diameter of rotary disk: 65 mm
Rotation speed of rotary disk: about 12,000 rpm
Feed (add) rate: about 8 kg/hour
Intake air temperature: about 50°C

Referential Example 2

Glycerin monostearate (213 parts by weight) was melted at 90-100°C. After confirmation of melting glycerin monostearate, ticlopidine hydrochloride (100 parts by weight) was added to the resultant liquid. The resultant mixture liquid was subjected to spray granulation by use of a spray drier to thereby obtain granules.

The spray conditions employed were identical to those employed in Example 6.

Evaluation

Each of granule samples of Example 6 and Referential Example 2 was put in a polymer-made sac and stored for one day. The flowability of the granule sample after storage was evaluated.

Table 4 shows the results.

Table 4

| | Flowability evaluation results |
|---|---|
| Ex. 6 | Caking generated, but no cakes of the size larger than 2 cm. Excellent flowability. |
| Ref. Ex. 2 | Caking generated with many cakes of the size larger than 2 cm. Poor flowability. |

Industrial Applicability

According to the present invention, there can be produced a granular pharmaceutical composition suitable for providing a pharmaceutical characterized in that adhesion of granules thereof onto a granulation apparatus during granulation is minimized and caking of the granules is suppressed.

## Claims

1. A pharmaceutical composition comprising a drug (A), a waxy substance (B), and synthetic aluminum silicate and/or hydrous silicon dioxide (C).

2. A pharmaceutical composition according to claim 1, which is a granular pharmaceutical composition.

3. A pharmaceutical composition according to claim 1 or 2, wherein the drug (A) has a disagreeable taste.

4. A pharmaceutical composition according to any one of claims 1 to 3, wherein the drug (A) is slightly soluble in the waxy substance.

5. A pharmaceutical composition according to any one of claims 1 to 3, wherein the drug (A) is soluble in water and slightly soluble in the waxy substance.

**6.** A pharmaceutical composition according to any one of claims 1 through 5, wherein the waxy substance (B) has a melting point of 40-150°C.

**7.** A pharmaceutical composition according to any one of claims 1 through 6, wherein the waxy substance (B) is at least one species selected from among hydrogenated oils, fats and oils of vegetable or animal origin, higher alcohols, polyethylene glycols, higher fatty acids, glycerin fatty acid esters, and sucrose fatty acid esters.

**8.** A pharmaceutical composition according to any one of claims 1 through 7, wherein the drug (A) is selected from among cetraxate hydrochloride, ecapapide, nefiracetam, talampicillin hydrochloride, indenolol hydrochloride, hydralazine hydrochloride, chloropromazine hydrochloride, tiaramide hydrochloride, berberine chloride, digitoxin, sulpyrine, azelastine hydrochloride, etilefurine hydrochloride, diltiazem hydrochloride, propranolol hydrochloride, chloramphenicol, aminophyllin, erythromycin, clarithromycin, phenobarbital, calcium pantothenate, indeloxazine hydrochloride, aminoguanidine hydrochloride, bifemelane hydrochloride, 7β-[2-(2-aminothiazol-4-yl)-2-(Z)-hydroxyiminoacetamido]-3-N,N-dimethylcarbamoyloxymethyl-3-cephem-carboxylic acid 1-(isopropoxycarbonyloxy)ethyl ester hydrochloride, (E)-3-(2-methoxy-3,6-dimethyl-1,4-benzoquinon-5-yl)-2-[5-(3-pyridyl)pentyl]-2-propenic acid, aminophylline, theophylline, diphenhydramine, metoclopramide, phenylbutazone, phenobarbital, ampicillin, cimetidine, famotidine, nizatidine, acetoaminophene, epirizol, pyrazinamide, caffeine, ethionamide, carbezirol, ranitidine hydrochloride, roxatidine acetate hydrochloride, imipramine hydrochloride, ephedrine hydrochloride, diphenhydramine hydrochloride, DONEPEDYL hydrochloride, tetracycline hydrochloride, doxycycline hydrochloride, naphazoline hydrochloride, noscapine hydrochloride, papaverine hydrochloride, dextrometorphan hydrobromide, timepidium bromide, chlorophenylammonium maleate, alimemazine tartrate, pilsicainide hydrochloride, N-methylscopolammonium methylsulfate, cinepazide maleate, arginine hydrochloride, hystidine hydrochloride, lysine hydrochlroride, lysine acetate; crude drugs or extracts thereof; pyrridonecarboxylic acid compounds represented by formulas (1) through (4) and salts thereof:

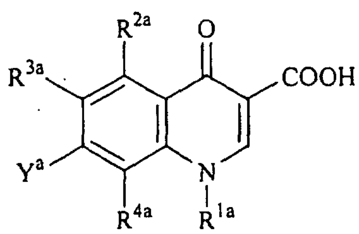

(1)

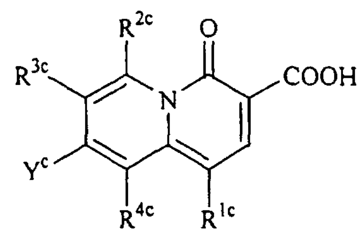

(2)

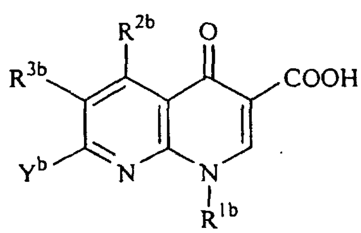

(3)

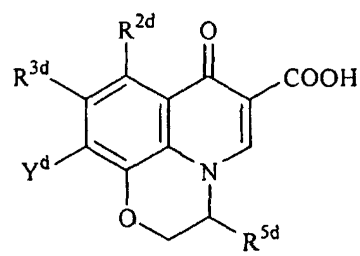

(4)

(wherein each of $R^{1a}$, $R^{1b}$, and $R^{1c}$ represents a C1-C6 linear or branched alkyl group which may have a substituent, a C3-C6 cyclic alkyl group which may have a substituent, an aryl group which may have a substituent, or a heteroaryl group which may have a substituent;

each of $R^{2a}$, $R^{2b}$, $R^{2c}$, and $R^{2d}$ represents a hydrogen atom or a C1-C6 linear or branched alkyl group which may have a substituent or an amino group;

each of $R^{3a}$, $R^{3b}$, $R^{3c}$, and $R^{3d}$ represents a hydrogen atom or a halogen atom;

$R^{4a}$ or $R^{4c}$ represents a hydrogen atom, a halogen atom, a C1-C6 linear or branched alkyl group which may have a substituent; or a C1-C6 linear or branched alkoxyl group which may have a substituent;

$R^{5d}$ represents a hydrogen atom or a C1-C6 linear or branched alkyl group which may have a substituent; and each of $Y^a$, $Y^b$, $Y^c$, and $Y^d$ represents a nitrogen-containing group); and 4,5,6,7-tetrahydrothieno[3,2-c]pyridines or salts thereof represented by formula (5):

$$R^1\text{-CH}(R^2)\text{-}R^3 \qquad (5)$$

[wherein $R^1$ represents a phenyl group which may have 1 to 3 substituents selected from the group consisting of a C1-C4 alkyl group, a halogen atom, a fluorine-substituted C1-C4 alkyl group, a C1-C4 alkoxy group, a fluorine-substituted C1-C4 alkoxyl group, a cyano group, and a nitro group;

$R^2$ represents a hydrogen atom, a carboxyl group, a C1-C6 alkoxycarbonyl group, or a C1-C7 aliphatic acyl group which may have a substituent selected from among a halogen atom, a hydroxyl group, a C1-C6 alkoxyl group, and a cyano group; and

$R^3$ represents a 4,5,6,7-tetrahydrothieno[3,2-c]pyridin-5-yl group which may have a substituent selected from among a hydroxyl group, a C1-C4 alkoxyl group, a C1-C4 alkoxyl group which are substituted by C1-C4 alkoxyl or C1-C6 alkanoyloxy, a C7-C14 aralkyloxy group, a C1-C18 alkanoyloxy group, a C3-C7 cycloalkylcarbonyloxy group, a C6-C10 arylcarbonyloxy group, a C1-C4 alkoxycarbonyloxy group, and a C7-C14 aralkyloxycarbonyloxy group].

**9.** A pharmaceutical composition according to any one of claims 1 through 8, wherein the drug (A) is ofloxacin, levofloxacin, ticlopidine hydrochloride, or clopidogrel sulfate.

**10.** A pharmaceutical composition according to any one of claims 1 through 9, which is prepared by melting the waxy substance by heating; dispersing or dissolving the drug, synthetic aluminum silicate, and/or hydrous silicon dioxide, and subjecting the resultant dispersion or solution to spray granulation.

**11.** A pharmaceutical composition according claim 10, which is prepared through further granulation by use of sugar alcohol.

**12.** A pharmaceutical product for oral administration, containing a pharmaceutical composition as recited in any one of claims 1 to 11.

**13.** A pharmaceutical product for oral administration according to claim 12, which has a drug form of powder, fine granules, or granules.

**14.** An agent for preventing adhesion of a granulated product onto the wall inside a granulation apparatus during spray granulation, the agent containing, as an effective ingredient, synthetic aluminum silicate or hydrous silicon dioxide.

## INTERNATIONAL SEARCH REPORT

International application No.
PCT/JP01/08137

A. CLASSIFICATION OF SUBJECT MATTER
Int.Cl$^7$ A61K9/16, 47/10, 47/14, 47/04, 31/4365, 31/497

According to International Patent Classification (IPC) or to both national classification and IPC

B. FIELDS SEARCHED

Minimum documentation searched (classification system followed by classification symbols)
Int.Cl$^7$ A61K9/16, 47/10, 47/14, 47/04, 31/4365, 31/497

Documentation searched other than minimum documentation to the extent that such documents are included in the fields searched

Electronic data base consulted during the international search (name of data base and, where practicable, search terms used)
CAPLUS (STN)

C. DOCUMENTS CONSIDERED TO BE RELEVANT

| Category* | Citation of document, with indication, where appropriate, of the relevant passages | Relevant to claim No. |
|---|---|---|
| PX | WO 00/054811 A1 (Daiichi Pharmaceutical Co., Ltd.), 21 September, 2001 (21.09.01), abstract; Claims; page 14, line 23 to page 18, line 7 & JP 2000-327590 A | 1-14 |
| PX | JP 2001-302497 A (Eisai Co., Ltd.), 31 October, 2001 (31.10.01), abstract; Claims; Par. Nos. [0014], [0018] (Family: none) | 1-14 |
| PX | JP 2001-270821 A (Eisai Co., Ltd.), 02 October, 2001 (02.10.01), abstract; Claims; Par. Nos. [0004], [0019] (Family: none) | 1-14 |
| PX | WO 00/59475 A1 (Lopocine, Inc.), 12 October, 2000 (12.10.00), Claims; page 14, line 20 to page 15, line 5 (Family: none) | 1-14 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 December, 2001 (13.12.01) | 25 December, 2001 (25.12.01) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No. PCT/JP01/08137

C (Continuation). DOCUMENTS CONSIDERED TO BE RELEVANT

| Category* | Citation of document, with indication, where appropriate, of the relevant passages | Relevant to claim No. |
|---|---|---|
| X | JP 9-95439 A (Eisai Co., Ltd.),<br>08 April, 1997 (08.04.97),<br>abstract; Claims; Par. Nos. [0007], [0015]<br>(Family: none) | 1-14 |
| X | JP 6-91150 A (Daiichi Pharmaceutical Co., Ltd.),<br>05 April, 1994 (05.04.94),<br>abstract; Claims; Par. Nos. [0008], [0010]<br>(Family: none) | 1-14 |
| X | JP 5-194193 A (Daiichi Pharmaceutical Co., Ltd.),<br>03 August, 1993 (03.08.93),<br>abstract; Claims; Par. Nos. [0006], [0007]<br>(Family: none) | 1-14 |
| X | JP 5-58880 A (Daiichi Pharmaceutical Co., Ltd.),<br>09 March, 1993 (09.03.93),<br>abstract; Claims; Par. Nos. [0008], [0011]<br>(Family: none) | 1-14 |
| X | WO 97/38960 A1 (Takeda Chemical Industries, Ltd.),<br>23 October, 1997 (23.10.97),<br>Claims; page 18, line 11 to page 19, line 10<br>& EP 902777 A1 & US 6235947 B1<br>& US 2001-001106 & JP 10-036291 A<br>& JP 2001-114712 A & JP 3126683 B<br>& CA 2251092 A & AU 9725222 A1<br>& AU 718658 B2 & CN 1221398 A<br>& BR 9708672 A & AT 202768 E<br>& ES 2158540 T3 & NO 9804803 A<br>& KR 2000005486 A & NO 2001002249 A | 1-14 |
| X | EP 729748 A1 (Nippon Shinyaku Company, Limited),<br>04 September, 1996 (04.09.96),<br>abstract; Claims; page 4, line 8 to page 6, line 15<br>& WO 95/13794 A1 & JP 2827511 B | 1-14 |

Form PCT/ISA/210 (continuation of second sheet) (July 1992)